# EUROPEAN PATENT APPLICATION

(11) **EP 2 565 188 A1**
(43) Date of publication of application: **06.03.2013**
(21) Application number: 11007052.1
(22) Date of filing: 30.08.2011
(51) Int. Cl.: C07D 305/14, A61K 31/337, A61P 35/00

(54) **Novel photoactivatable paclitaxel derivatives and uses thereof, in particular in cellbiology and cancer treatment**

(71) Applicant: Max-Planck-Gesellschaft zur Förderung der Wissenschaften e.V., 80539 München (DE); Universitätsmedizin der Johannes Gutenberg-Universität Mainz, 55131 Mainz (DE)
(72) Inventor: del Campo Bècares, Aranzazu, 55128 Mainz (DE); Gropenau, Radu, Adrian, 55246 Mainz-Kostheim (DE); Ritz, Sandra, 55262 Heidesheim am Rhein (DE); Mailaender, Volker, 55122 Mainz (DE)
(74) Representative: Katzameyer, Michael

(57) **Abstract**

The present invention provides novel photoactivatable paclitaxel derivatives wherein at least the C7 position, preferably both the C2' and C7 positions, of the paclitaxel molecule is/are substituted with a photocleavable moiety such as a chromophore.

Typically, the chromophore is selected from the group comprising 2-nitrobenzyl ethers, carbamates or carbonates and their 4,5-dialkoxy substituted variants; 2-nitrophenylethyl ethers, carbamates or carbonates and their 4,5-dialkoxy substituted variants, or their p-substituted derivatives; α-carboxy-2-nitrobenzyl ethers, carbamates or carbonates and their 4,5-dialkoxy substituted variants; 2(2-nitrophenyl)ethyl ethers, carbamates or carbonates and their 4,5-dialkoxy substituted variants; coumarin-4-ylmethyl ethers, or carbonates and their 7-alkoxy, 6,7-dialkoxy, 6-bromo-7-alkoxy or 7-dialkylamino substituted variants; p-hydroxyphenacyl ethers or carbamates and their meta-substituted derivatives or structurally related compounds.

More specifically, the chromophore is 4,5-dimethoxy-2-nitrobenyloxycarbonyl (Nvoc) or a structurally related compound.

Further aspects of the invention relate to the use of these paclitaxel derivatives in biological, medical and pharmaceutical applications, in particular in cellbiology and cancer treatment. A closely related aspect is an antitumor medicament comprising the above paclitaxel derivatives.

## Description

### Background

Paclitaxel (PTX, Taxol®) is one of the most important antitumor drugs clinically used for cancer treatment. The bioactivity of paclitaxel is intimately connected to its molecular interaction with tubulin. Paclitaxel promotes tubulin assembly into stable, aggregated structures that resist depolymerisation by dilution, calcium ions, cold or other microtubule-disrupting drugs. The alteration of the microtubule dynamics causes an extended mitotic arrest that eventually leads to cell death. PTX also finds extensive in-vitro applications, i.e. as microtubule stabilisator for studying the interaction between motor proteins and other microtubule associated proteins (MAPs), or for directing microtubule growth and therefore molecular transport in bionanotechonology approaches (see Diez et al., Current Opinion in Biotechnology 2010, 21, 477).

Many different variants of Taxol have been synthesized since the 1980s, seeking for higher activity and selectivity towards tumors and less toxicity towards normal tissue (see Fu et al., Current Medicinal Chemistry 2009, 16, 3966, for a recent review) and for improving its poor solubility (via BSA or polymer conjugates) or pharmacokinetics (reviewed in R. Haag, F. Kratz, Angewandte Chemie-International Edition 2006, 45, 1198) for clinical applications. Fluorescent PTX derivatives have also been reported (e.g. Diaz et al. in Journal of Biological Chemistry 2005, 280, 3928) and applied to imaging microtubule formation in *in-vitro* experiments. Some photosensitive derivatives of PTX have been proposed for light-controlled delivery of PTX and photodynamic therapy, but neither properties nor applications of the compounds have been described (M. Noguchi et al., Bioorganic & Medicinal Chemistry 2008, 16, 5389; M. Skwarczynski et al., Bioorganic & Medicinal Chemistry Letters 2006, 16, 4492).

A photoactivatable caged derivative of a bioactive molecule allows dynamic control of its bioactivity by light exposure. The "cage" is typically a chromophore unit which is (mostly) covalently bounded to the active site of the molecule and inhibits its bioactivity. Upon light exposure the chromophore is cleaved from the structure and the activity of the biomolecule is restored. Using flash irradiation and focused lasers, the presence and concentration of the active biomolecule can be regulated with accurate spatiotemporal resolution (down to submicrometric and microsecond scales). This approach has been applied to phototrigger e.g. ATP, glutamate and Ca²⁺ concentration jumps in cell biology experiments (M. Goeldner, R. Givens (Eds.), Dynamic studies in Biology, Wiley-VCH 2005).

A photoactivatable caged taxol was commercially available from Molecular Probes and Invitrogen until 2003 and used in a few reported cell biology studies for controlled microtubule growth (F. Bradke, H. Witte, D. Neukirchen, Journal of Cell Biology 2008, 180, 619; B. Buck, J. Q. Zheng, Journal of Neuroscience 2002, 22, 9358). However, this caged taxol, 2'-Nvoc-PTX, was retracted from the market in 2003. One possible reason may be insufficient inhibition of bioactivity in the caged format and/or its cytotoxicity at typical concentrations for in-vivo and in-vitro applications.

Thus, a main object of the present invention is to provide improved photoactivatable paclitaxel derivatives having a considerably reduced cytotoxicity and biological activity in the "caged" state but can be activated in a simple and effective manner by irradiation with light.

This object is achieved by providing the photoactivatable paclitaxel derivatives according to claim 1 and the use of said derivatives according to claim 10. Additional aspects and/or preferred embodiments of the present invention are the subject of further claims.

### Description of the invention

The present invention provides novel photoactivatable paclitaxel derivates wherein at least the C7 position of the paclitaxel molecule is substituted with a photocleavable moiety. Preferably, the paclitaxel derivatives are disubstituted at the C7 and C2' positions with photocleavable moieties which may be the same or different.

Preferably, the photo-cleavable moiety is cleavable by means of irradiation with light having a wavelength > 320 nm, in particular in the range from 320 to 500 nm for single photon excitation processes and > 500 nm for multiphoton excitation.

Typically, the photocleavable moiety or moieties is/are a chromophore, in particular belonging to the following families or classes (see Scheme 1 below):
- 2-nitrobenzyl ethers, carbamates or carbonates and their 4,5-dialkoxy (nitroveratryl) substituted variants
- 2-nitrophenylethyl ethers, carbamates or carbonates and their 4,5-dialkoxy substituted variants
- α-carboxy-2-nitrobenzyl ethers, carbamates or carbonates and their 4,5-dialkoxy substituted variants
- 2(2-nitrophenyl)ethyl ethers, carbamates or carbonates and their 4,5-dialkoxy substituted variants
- coumarin-4-ylmethyl ethers, or carbonates and their 7-alkoxy, 6,7-dialkoxy, 6-bromo-7-alkoxy or 7-dialkylamino substituted variants
- p-hydroxyphenacyl ethers or carbamates and their meta-substituted derivatives
or structurally related compounds.

Suitable chromophores for single photon excitation processes can be selected from these classes of compounds.

Alternatively, chromophores with sufficient multiphoton absorption cross-section can also be used like 2-nitrophenylethyl ethers, carbonates or carbamates or their p-substituted (OR, NR₂) derivatives (see Scheme 1 below).

More specifically, the chromophore(s) is/are 4,5-dimethoxy-2-nitrobenyloxycarbonyl (Nvoc) or a structurally related compound. 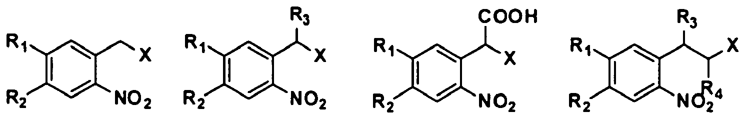 R₁, R₂ = H, CH₃, O(CH₂)nCH₃, O(CH₂CH₂)nOCH₂CH₃
R₃, R₄ = H, CH₃, (CH₂)n)CH₃
X= O, OCOO, OCONH 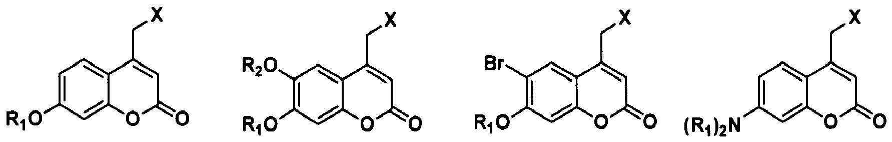 R₁ = H, CH₃, OH, O(CH₂)nCH₃, O(CH₂CH₂)nOCH₂CH₃, O(CH₂)nCOOH
R₂ = H, CH₃, O(CH₂)nCH₃, O(CH₂CH₂)nOCH₂CH₃, O(CH₂)nCOOH
X= O, OCOO, OCONH 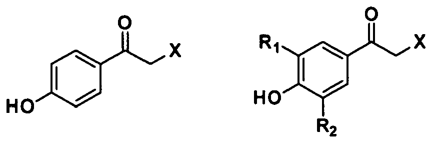 R₁, R₂ = H, OCH₃, COHN₂, COOCH₃, COOH
X = O, OCOO 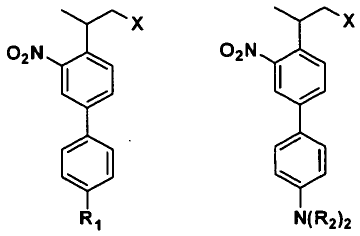 R₁ = H, CH₃, OCH₃, O(CH₂)nCH₃, O(CH₂CH₂O)CH₂CH₃
R₂ = H, CH₃, OCH₃, O(CH₂)nCH₃, O(CH₂CH₂O)CH₂CH₃, COOH
X = O, OCOO, OCONH
with n in each of the above structural formulae independently being an integer from 1 to 50, preferably 1 to 30, such as 1-20 or 2-20

**Scheme 1.** Compilation of exemplary chromophores for use as photocleavable moieties in the paclitaxels derivatives of the invention

The structure of the base molecule PTX (1) and the numbering is presented in Formula 1 below.

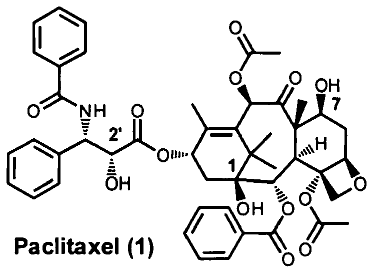

It is generally accepted that the C13 (2'R, 3'S)-Nbenzoyl-3'-phenylisoserine side-chain is essential for cytotoxicity of paclitaxel (Fu et al., Current Medicinal Chemistry 2009, 16, 3966). There are three reactive hydroxyl groups in positions C2', C1 and C7 that can be used for derivatization. The reactivity of this positions towards esterification follows the sequence C2'>C7>C1. According to published literature on PTX derivatives, substitution at C1 does not significantly affect activity of PTX. Esterification at C7 usually resulted in loss of *in vitro* microtubule assembly activity while cytotoxicity was maintained. (Fu et al., Current Medicinal Chemistry 2009, 16, 3966). This position has often been used to synthesize prodrugs of paclitaxel with improved water solubility. Esterification at C2' usually resulted in loss of microtubule disassembly activity in vitro, but not cytotoxicity. (Fu et al., Current Medicinal Chemistry 2009, 16, 3966).

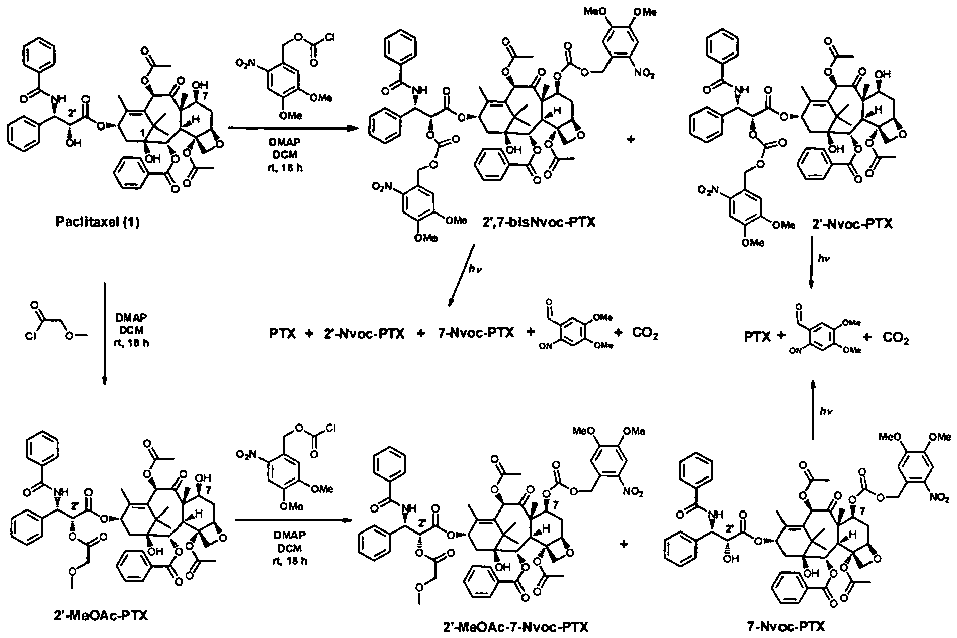

**Scheme 2.** Synthesis and photolysis of photocleavable caged Paclitaxel derivatives

The present inventors synthesized three different caged variants of PTX according to Scheme 2 above by attaching the photoremovable chromophore 4,5-dimethoxy-2-nitrobenzyloxycarbonyl (Nvoc) at position C7, C2' and at both of them via reaction with Nvoc-C1 and formation of a carbonate bond (Example 1). Mass spectrometry and the appearance of the characteristic signals for the Nvoc unit in the NMR spectra confirmed the molecular structure of the substituted PTX. 2' substitution shifted the C2' proton signal in the ¹H-NMR spectrum from 4.8 to 5.6 ppm, in agreement with other reported C2' substituted PTX derivatives (Greenwald et al., Journal of Medicinal Chemistry 1996, 39, 424). For the synthesis of 7-Nvoc-PTX, the 2'-OH group was first protected as methoxyacetate ester (Greenwald et al., Journal of Organic Chemistry 1995, 60, 331) before reaction with NVoc-Cl and deprotection in basic conditions. The chemical shift of the C7 proton shifted from 3.78 to 5.5 ppm upon Nvoc functionalization. It is important to note that 7-Nvoc-PTX could also be obtained as photolysis product of 2',7-bisNvoc-PTX. All caged derivatives of PTX were stable as solid and as DMSO solution. No hydrolysis was detected upon storage in 80 mM PIPES buffer (pH 6.8) and in dark over 4 weeks. Despite the fact that the Nvoc substitution increased the hydrophobicity of the PTX moiety, the caged PTX derivatives have a slightly better water solubility than PTX. In fact, a 10 mM DMSO solution could be diluted with PIPES buffer to 1:1000 (Table A in Example 2).

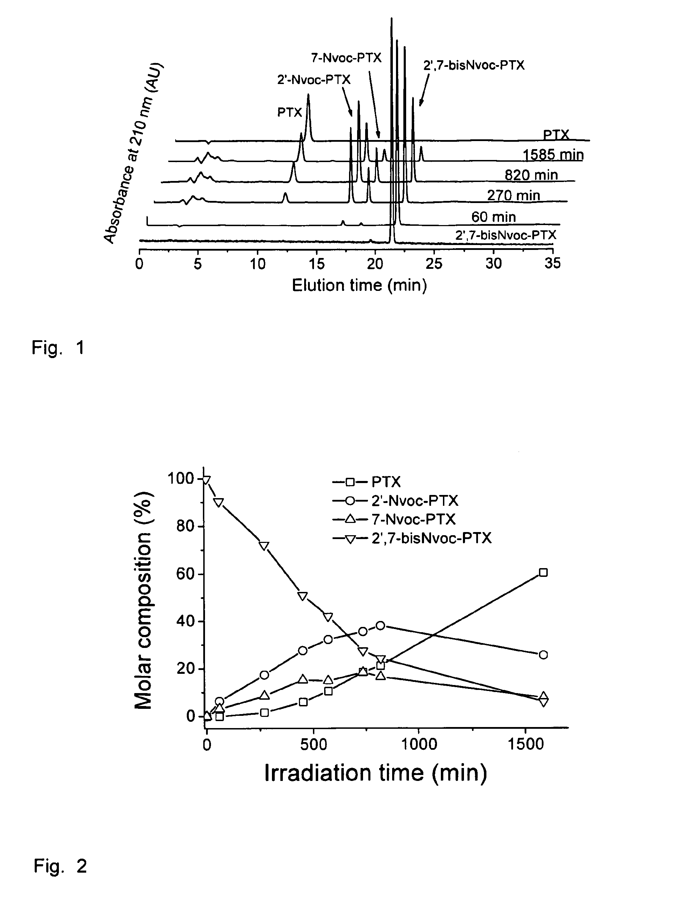

The photolysis of the compounds 2'-Nvoc-PTX and 2',7-bisNvoc-PTX was followed by UV and HPLC analysis of the correspondent solutions after exposure to light at λ*ₘₐₓ* = 360 nm. As expected, photocleavage of compound 2',7-bisNvoc-PTX yields the monosubstituted species 2'-Nvoc-PTX and 7-Nvoc-PTX as intermediates that can be further photolyzed to afford free PTX. In fact, HPLC analysis (Fig. 1) confirmed the presence of 2'-Nvoc-PTX and 7-Nvoc-PTX together with the starting compound 2',7-bisNvoc-PTX and the fully deprotected PTX. The ratio of these compounds within the composition of the irradiated mixture changes with the exposure dose. Fig. 2 (and Table B in Example 2) shows the compositional evolution of the mixture with increasing irradiation time as obtained by HPLC analysis. The photocleavage of the carbamate at position 7 seems to be more effective than at position 2' and higher amounts of 2'-Nvoc-PTX than of 7-Nvoc-PTX were present in the mixture at all irradiation times.

In order to evaluate the bioactivity of the caged PCT derivatives, their cytotoxicity was tested *in vitro* using HeLa cells. Cells were incubated with PTX and caged PTX derivatives at concentrations between 0.01 and 10 µM. After a suitable time period of cultivation, usually 48 h, cells were stained with propidium iodide and the number of cells in the wells was counted. The vitality of the cells (%) is the ratio between dead cells and the total cell population per growing area. A vitality ~1 means no cytotoxicity, whereas vitalities <1 indicates cytotoxic effects.

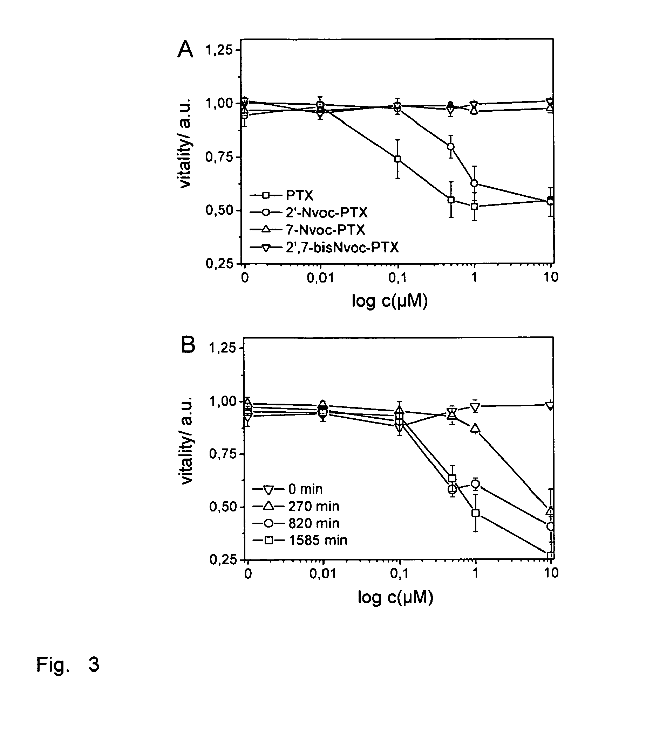

Fig. 3 shows the vitality for the PTX derivatives at different concentrations. In the positive control, PTX significantly reduced cell vitality at a concentration of 0.1 µM. This value agrees with reported cytotoxicity levels by other groups (Ceruti et al., Journal of Controlled Release 2000, 63, 141). Interestingly, 7-Nvoc-PTX and 2',7-bisNvoc-PTX did not show any cytotoxicity at concentrations at least up to 10 µM, indicating the cage group effectively inhibited the activity of the drug. 2'-Nvoc-PTX showed a ~25% reduction in vitality at 0.5 µM, indicating a reduced but still significant cytotoxicity in spite of the caged 2' position. The vitality results demonstrate that 2'-Nvoc-PTX can only be regarded as caged PTX over a very narrow concentration range (<0.1 µM), whereas the new derivatives worked over the whole range of concentrations tested (up to 10 µM).

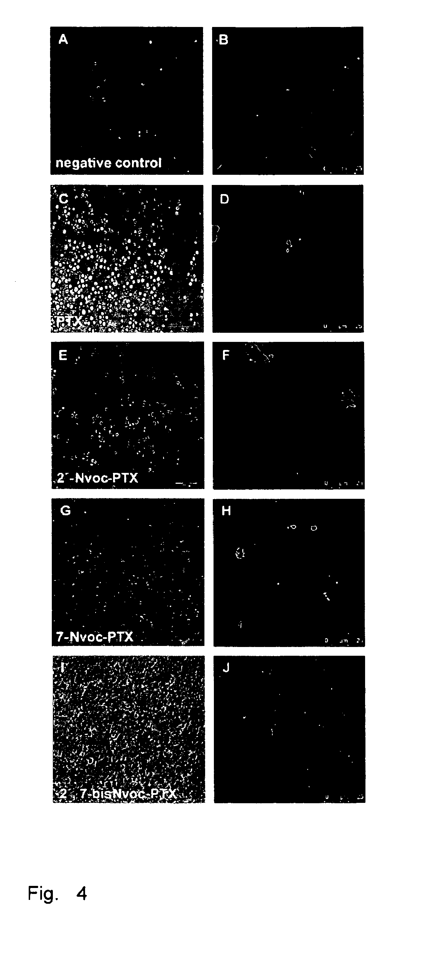

In order to visualize the influence of the PTX derivatives in the microtubule network of the cell, a live cell microscopy analysis of HeLa cells after incubation with 10 µM PTX derivatives for 24 hours (see Fig. 4 and Example 2 for details). The results agree with the vitality tests and demonstrate that the bisubstituted 2',7-bisNvoc-PTX effectively inhibited binding of PTX to β-tubulin at concentrations up to 10 µM, whereas the monosubstituted derivative 2'-Nvoc-PTX and 7-Nvoc-PTX were not able to completely inhibit PTX bioactivity. In fact, 2'-Nvoc-PTX already bound to tubulin at concentrations > 10 nM and 7-Nvoc-PTX bound to tubulin at concentrations > 1 µM. Thus, these results indicate that caging at both positions (2' and 7) appears to be necessary in order to fully inhibit PTX activity and to obtain a fully working PTX phototrigger over a very broad concentration range. Nevertheless, 7-Nvoc-PTX still represents a major improvement over compounds of the prior art such as 2'-Nvoc-PTX.

As demonstrated above, it is possible by substituting both the 2' and 7 positions of PTX to obtain paclitaxel derivatives which have essentially no cytotoxicity up to a relatively high concentration of at least 1 µM, preferably at least 10 µM.

Thus, in a specific embodiment of the invention, the claimed paclitaxel derivatives are characterized by having no cytotoxicity up to a concentration of at least 10 µM.

In a further specific embodiment of the invention, the claimed paclitaxel derivatives have a reduced ß-Tubulin binding activity which is not more than about 1 %, preferably about 0.1%, in particular not more than about 0.02% or 0.002% of the activity of underivatized paclitaxel for the 7- and 2',7-bisubstituted derivatives respectively.

Due to their favourable characteristics, the paclitaxel derivatives of the invention may be, e.g., advantageously used in the fields of medicine and cell biology.

Thus, a further aspect of the present invention relates to the use of these paclitaxel derivatives in biological, medical and pharmaceutical applications.

More specifically, these paclitaxel derivatives may be used for microtubule stabilisation and affecting and/or monitoring/detecting the interaction between motor proteins and other microtubule associated proteins, or for directing microtubule growth and microtuble-related molecular transport in bionanotechnology approaches.

Also, these paclitaxel derivatives may be used for preparing a medicament for treating tumors, in particular in photodynamic tumor therapy.

Some non-limiting examples for such tumors are solid tumors, such as metastatic breast cancer, head and neck carcinomas, ovarian carcinoma, non-small cell lung cancer, melanoma, Kaposi's sarcoma etc.

A closely related aspect of the present invention relates to an antitumor medicament comprising the paclitaxel derivative according to any one of claims 1-9. More specifically, this antitumor medicament is for treating solid tumors, such as metastatic breast cancer, head and neck carcinomas, ovarian carcinoma, non-small cell lung cancer, melanoma, Kaposi's sarcoma etc.

The invention is further illustrated by the following non-limiting Examples and Figures.

### FIGURES

**Fig. 1****.** HPLC curves of a 1 mM solution of 2',7-bisNvoc-PTX after irradiation at 360 nm with increasing dose showing the appearance of the monocaged derivatives and free PTX.
**Fig. 2****.** Quantification of the HPLC data in terms of molar composition of the solution.
**Fig. 3****.** Evaluation of the cytotoxicity of PTX derivatives versus concentration. **A)** vitality of three caged PTXs derivatives compared to free PTX. **B)** Vitality of cells after incubation with irradiated solutions of 2',7-bisNvoc-PTX containing different amounts of uncaged (free) PTX.
**Fig. 4****.** Bright field (20x) and fluorescence CLSM images (100x) of living HeLa cells treated with 10 µM PTX derivatives for 24 h: (A, B) negative control, (C, D) PTX, (F, G) 2'-Nvoc-PTX, (H, I) 7-Nvoc-PTX, (J, K) 2',7-bisNvoc-PTX.
**Fig. 5** Concentration dependent appearance of multiple nuclei induced by PTX and single caged compounds. Fluorescence CLSM images (1000x) of living HeLa cells treated for 24 h with 0.2 to 100 nM concentrations of PTX, 2'-Nvoc-PTX and 7-Nvoc-PTX. Microtubuli were stained with Tubulin Tracker (green) and nuclei with DRAQ5 (blue).
**Fig. 6** Concentration dependent appearance of multiple nuclei induced by PTX and single caged compounds. Fluorescence CLSM images (1000x) of living HeLa cells treated for 24 h with (A) 2 nM PTX, (B) 50nM 2'-Nvoc-PTX and (C) 10 µM 7-Nvoc-PTX. Microtubuli were stained with Tubulin Tracker (green) and nuclei with DRAQ (blue).

### EXAMPLE 1

### Synthesis of paclitaxel derivatives

### 1. Materials and Equipment

Paclitaxel (Xingcheng Chempharm Ltd., Taizhou, China), Nitroverantryl chloride (Nvoc-Cl, Merck, Darmstadt, Germany), dimethylamino pyridine (DMAP), methoxy-acetyl chloride and dry dichloromethane (Acros, Belgium) were purchased as p.a. quality and used without other purifications. The reactions course was followed using silicagel and RP18-silicagel TLC plates (Merck, Darmstadt, Germany).

NMR spectra were measured at room temperature with Brucker Avance 300 spectrophotometer. UV-Vis spectra were recorded with a Varian Cary 4000 UV/VIS spectrometer. Irradiation experiments were performed with a LUMOS 43 (Atlas Photonics Inc.) operating at 360 nm. PTX derivatives were purified on a JASCO HPLC 2000 series equipped with a diode array UV-Vis detection system and fraction collector, using a semi-preparative column (250 × 20 mm) filled with Reprosil RP18 (5 µm grain size). For the analytical measurements, a smaller column (250 × 5 mm) filled with the same material was used.

### 2. Synthesis of 2'-Nvoc-PTX and 2',7-bisNvoc-PTX

A solution of 500 mg PTX (585 µmol, 1 equiv.), 242 mg Nvoc-Cl (880 µmol, 1.5 equiv.) and 107 mg DMAP (880 µmol, 1.5 equiv.) in 20 ml dry dichloromethane (DCM) was stirred overnight under inert atmosphere in dark at r.t. After 18 hrs, additional 100 ml DCM and 200 ml water were added to the reaction mixture. The organic phase was washed with water (2 × 100 ml), dried with magnesium sulfate and the solvent was removed in vacuum. The yellow residue was dissolved in acetonitril containing 5% water and the products were separated by HPLC (solution A: water + 0.1% TFA, solution B: acetonitril + 5% water + 0.1% TFA; gradient from 50% B to 100% B in 25 min). Traces of PTX, 406 mg of 2'-Nvoc-PTX (yield 64%) and 156 mg of 2',7-bisNvoc-PTX (yield 21%) were isolated.

**2'-Nvoc-PTX:** ¹H-NMR (CD₂Cl₂, 300 MHz) : δ (ppm) 1.12 (3H, s); 1.23 (3H, s); 1.77-1.80 (4H, m); 2.15-2.26 (4H, m); 2.37-2.43 (1H, m); 2.46 (3H, s); 2.50-2.58 (1H, m); 3.80 (1H, d, J = 6 Hz) ; 3.86 (3H, s, -OC*H*₃) ; 3.91 (3H, s); 4.19 (1H, d, J = 6 Hz); 4.32 (1H, d, J = 6 Hz); 4.43 (1H, dd, J^{a} = 9 Hz, J^{b} = 6 Hz); 5.00 (1H, dd, J^{a} = 3 Hz, J^{b} = 9 Hz); 5.48 (1H, d, J = 3 Hz); 5.60 (2H, 2xd, J^{gem} = 15 Hz); 5.67 (1H, d, J = 3 Hz); 6.01 (1H, dd, J^{a} = 3 Hz, J^{b} = 9 Hz); 6.24-6.29 (2H, m); 6. 96 (1H, s); 7.08 (1H, d, J = 12 Hz); 7.36-7.46 (7H, m); 7.52-7.57 (3H, m); 7.60-7.67 (1H, m); 7.71-7.74 (3H, m); 8.13-8.15 (2H, m). ¹³C-NMR (CD₂Cl₂, 75 MHz): δ (ppm) 10.02; 15.10; 21.20; 22.51; 23.22; 27.14; 36.05; 43.78; 46.25; 56.92; 68.03; 72.77; 72.91; 75.65; 76.15; 77.59; 79.63; 81.53; 85.06; 108.92; 110.39; 127.22; 127.68; 129.28; 129.32; 129.72; 129.88; 130.72; 132.85; 133.59; 134.27; 136.98; 143.06; 149.26; 154.53; 154.61; 167.42; 167.79; 168.53; 168.62; 170.56; 172.00 204.30. FD-MS: 1093.1 (M+1)⁺.

**2',7-bisNvoc-PTX:** ¹H-NMR (CD₂Cl₂, 300 MHz) : δ (ppm) 1.15 (3H, s); 1.21 (3H, s); 1.80 (3H, s); 1.95-2.01 (5H, m); 2.08 (3H, s); 2.20-2.28 (1H, m); 2.39-2.47 (1H, m); 2.49 (3H, s); 2.56-2.67 (1H, m); 3.86 (3H, s, -OC*H*₃); 3.91-3.99 (10H, m); 4.17 (1H, d, J = 6 Hz); 4.34 (1H, d, J = 9 Hz) ; 5.00 (1H, d, J = 9 Hz); 5.47-5.69 (7H, m); 6.02 (1H, dd, J^{a} = 3 Hz, J^{b} = 6 Hz); 6.23 (1H, t, J = 9 Hz); 6.33 (1H, s); 6.97 (1H, s); 7.01 (1H, d, J = 9 Hz); 7.09 (1H, s); 7.37-7.46 (7H, m); 7.50-7.57 (3H, m); 7.62-7.67 (1H, m); 7.70 (1H, s); 7.71-7.75 (3H, m); 8.14-8.17 (2H, m). ¹³C-NMR (CD₂Cl₂, 75 MHz): δ (ppm) 11.19; 14.80; 21.03; 21.71; 23.24; 26.75; 33.86; 36.01; 43.84; 47.63; 56.63; 56.90; 56.98; 67.18; 67.99; 72.78; 75.00; 75.86; 76.45; 79.26; 81.33; 84.29; 108.65; 108.90; 110.43; 111.14; 125.15; 126.43; 127.24; 127.34; 127.63; 129.11; 129.23; 129.30; 129.68; 129.83; 130.74; 132.59; 132.68; 133.33; 134.28; 137.27; 138.71; 140.14; 140.30; 141.50; 141.66; 143.49; 148.98; 149.23; 153.18; 154.31; 154.42; 154.58; 165.69; 167.30; 167.79; 168.56; 168.64; 169.62; 170.61; 204.13. FD-MS: 1332.0 (M+1)⁺.

### 3. Synthesis of 7-Nvoc-PTX

A solution of 63 mg methoxyacetyl chloride (58 µl, 550 µmol, 1.1 equiv.) in 1 ml dry DCM was added dropwise to a solution of 426 mg PTX (500 µmol, 1 equiv.), 67 mg DMAP (600 µmol, 1.2 equiv.) in 10 ml dry DCM and the reaction mixture was stirred overnight under inert atmosphere in dark at r.t. After 18 hrs, additional 100 ml dichloromethane and 100 ml water were added to the reaction mixture and stirred for 15 at r.t. The organic phase was separated, washed with water (2 × 100 ml), dried with magnesium sulfate and the solvent was removed in vacuum. The white residue was dissolved in acetonitril containing 5% water and the products were separated by HPLC (solution A: water + 0.1% TFA, solution B: acetonitril + 5% water + 0.1% TFA; gradient from 20% B to 100% B in 25 min). 340 mg of 2'-MeOAc-PTX (yield 67%) were isolated.

¹H-NMR (CD₂Cl₂, 300 MHz): δ (ppm) 1.13 (3H, s); 1.22 (3H, s); 1.64 (3H, s); 1.75-1.85 (1H, m); 1.94 (3H, s); 1.96 (1H, s); 2.18-2.27 (7H, m); 2.37-3-2.54 (6H, m); 2.69 (1H, bs); 3.35 (3H, s, 2'-C*H*₃-O-CH₂); 3.82 (1H, d, J = 6 Hz); 4.13 (2H, dd, J = 12 Hz, 2'-CH₃-O-C*H*₂-) ; 4.17 (1H, d, J = 9 Hz), 4.29 (1H, d, J = 9 Hz), 4.98 (1H, d, J = 9 Hz); 5.60 (1H, d, J = 3 Hz) ; 5.67 (1H, d, J = 6 Hz); 6.03 (1H, dd, J^{a} = 3 Hz, J^{b} = 6 Hz); 6.23 (1H, t, J = 9 Hz); 6.30 (1H, s); 7.32-7.45 (8H, m); 7.49-7.57 (3H, m); 7.62-7.66 (1H, m); 7.74-7.77 (2H, m); 8.14-8.17 (2H, m). ¹³C-NMR (CD₂Cl₂, 75 MHz): δ (ppm) 10.02; 15.02; 21.16; 22.48; 23.16; 27.09; 36.20; 43.74; 46.28; 53.28; 58.90; 59.74; 69.91; 72.56; 72.70; 74.77; 75.66; 76.12; 76.85; 79.36; 81.52; 84.86; 127.24; 127.67; 128.98; 129.15; 129.22; 129.56; 130.03; 130.69; 132.48; 133.55; 134.12; 134.37; 137.44; 142.90; 167.22; 167.97; 168.50; 170.19; 170.52; 171.75; 204.13. FD-MS: 926.8 (M+1)⁺.

A solution of 100 mg 2'-MeOAc-PTX (108 µmol, 1 equiv.), 44 mg Nvoc-Cl (162 µmol, 1.5 equiv.) and 20 mg DMAP (170 µmol, 1.6 equiv.) in 10 ml dry dichloromethane (DCM) was stirred overnight under inert atmosphere in dark at r.t. After 18 hrs, additional 100 ml DCM and 100 ml water were added to the reaction mixture. The organic phase was separated, washed with water (2 × 100 ml), dried with magnesium sulfate and the solvent was removed in vacuum. The yellow residue was dissolved in acetonitril containing 5% water and the products were separated by HPLC (solution A: water + 0.1% TFA, solution B: acetonitril + 5% water + 0.1% TFA; gradient from 50% B to 100% B in 25 min). 19 mg PTX1, 38 mg 2'-MeOAc-PTX, 13 mg 7-Nvoc-PTX (yield 19%) and 17 mg of 2'-MeOAc-7-Nvoc-PTX (yield 23%) were isolated. According to literature reports, the MeOAc group is fair stability in acidic and neutral media and can be selectively removed by basic treatment without disturbing the other esters of the PTX molecule (Greenwald et al., Journal of Organic Chemistry 1995, 60, 331).

**2'-MeOAc-7-Nvoc-PTX:** ¹H-NMR (CD₂Cl₂, 300 MHz): δ (ppm) 1.15 (3H, s); 1.20 (3H, s); 1.80 (3H, s); 1.91-1.2.03 (2H, m); 2.08 (3H, s); 2.18-2.27 (1H, m); 2.37-2.42 (1H, m); 2.48 (3H, s); 2.58-2.68 (1H, m); 3.37 (3H, s, 2'-C*H*₃-O-CH₂); 3.92 (3H, s, - *OCH₃);* 3.95-4.01 (1H, m); 3.99 (3H, s, -OC*H*₃); 4.06-4.20 (3H, m); 4.34 (1H, d, J = 9 Hz); 4.99 (1H, d, J = 9 Hz); 5.48-5.69 (5H, m); 5.99 (1H, dd, J^{a} = 3 Hz, J^{b} = 6 Hz); 6.21 (1H, t, J = 7.5 Hz) ; 6.34 (1H, s); 7.06 (1H, d, J = 9 Hz); 7.10 (1H, s); 7.32-7.47 (7H, m); 7.50-7.57 (3H, m); 7.62-7.67 (1H, m); 7.71 (1H, s); 7.73-7.76 (2H, m); 8.13-8.16 (2H, m). ¹³C-NMR (CD₂Cl₂, 75 MHz): δ (ppm) 11.20; 14.81; 21.03; 23.20; 26.77; 33.88; 36.07; 43.82; 46.63; 56.88; 56.99; 59.82; 67.18; 69.91; 74.60; 84.28; 108.67; 111.17; 127.26; 127.62; 129.24; 129.31; 129.62; 130.74; 137.44; 141.81; 148.99; 154.30; 154.44; 167.31; 167.57; 168.56; 169.61; 170.16; 170.60; 202.51. FD-MS: 1166.1 (M+1)⁺

**7-Nvoc-PTX:** ¹H-NMR (CD₂Cl₂, 300 MHz): δ (ppm) 1.15 (3H, s); 1.20 (3H, s); 1.80 (3H, s); 1.87 (3H, s); 1.93-2.01 (2H, m); 2.08 (3H, s); 2.32-2.41 (5H, m); 2.56-2.66 (1H, m); 3.65 (1H, s); 3.92-3.95 (4H, m); 3.99 (3H, s, -OC*H*₃) ; 4.15 (1H, d, J = 9 Hz) ; 4.32 (1H, d, J = 9 Hz); 4.81 (1H, d, J = 1 Hz); 4.98 (1H, d, J = 9 Hz); 5.48-5.53 (2H, m); 5.62-5.68 (2H, m); 5.77 (1H, dd, J^{a} = 3 Hz, J^{b} = 6 Hz); 6.18 (1H, t, J = 9 Hz); 6.31 (1H, s); 7.08 (1H, d, J = 9 Hz); 7.10 (1H, s); 7.33-7.56 (10H, m); 7.62-7.67 (1H, m); 7.71 (1H, s); 7.74-7.77 (2H, m); 8.13-8.15 (2H, m). ¹³C-NMR (CD₂Cl₂, 75 MHz): δ (ppm) 11.17; 15.02; 21.05; 26.85; 33.25; 36.26; 43.80; 47.69; 56.76; 56.86; 56.9; 67.19; 72.83; 73.90; 79.15; 81.45; 84.23; 108.67; 111.06; 127.36; 127.59; 127.65; 129.22; 130.72; 132.45; 133.58; 134.32; 134.45; 138.92; 140.29; 141.24; 149.01; 154.35; 154.44; 167.20; 167.67; 169.66; 171.24; 173.17; 202.40. FD-MS: 1094.0 (M+1)⁺

### EXAMPLE 2

### Characterization and testing of paclitxel derivatives

### 1. Solubility tests

10 mM solutions of PTX derivatives in DMSO were prepared. All substances were soluble in DMSO at this concentration. 10 µM solutions of were obtained by 1:1000 dilution of the stock solution into 80 mM PIPES buffer pH 6.8, containing 2 mM MgCl₂ and 1 mM EGTA.

**Table A: solubility test of 10 µM solutions of PTX derivatives in 1:1000 DMSO:PIPES buffer**

| Time | PTX | 2'-Nvoc-PTX | 2',7-bisNvoc-PTX |
|---|---|---|---|
| 5 min | clear solution | clear solution | clear solution |
| 1 hr | clear solution | clear solution | clear solution |
| 8 hrs | clear solution | clear solution | clear solution |
| 24 hrs | Suspension | turbid solution | clear solution |
| 30 hrs | white precipitate | Suspension | turbid solution |

### 2. Photocleavage studies

The photolysis of the compounds 2'-Nvoc-PTX and 2',7-bisNvoc-PTX was followed by UV and HPLC analysis of the correspondent solutions after exposure to light at λ*ₘₐₓ* = 360 nm (2.7 mW/cm²).

3 ml of a 1 mM solution of caged PTX in acetonitrile containing 5% water was placed into a quartz cuvette and was exposed at 360 nm for different irradiation times. Aliquots of 50 µl were taken after various irradiation times, and each aliquot was splitted in two parts. 20 µl were diluted to 400 µl to obtain 50 µM solution for UV-Vis characterisation. The 30 µl left from each aliquot were injected into the HPLC (20 µl loop, analytical column) in order to estimate the composition of the irradiated mixture.

UV spectra of the irradiated solutions showed hypsochromic and hypochromic shifts of the absorption maxima and the appearance of a maximum with low absorbance at 396 nm (data not shown). These changes agree with reported data on the photocleavage of Nvoc derivatives and formation of the nitroso product during photolysis as a result of an intramolecular redox reaction (a) V. San Miguel-Arnanz, C. G. Bochet, A. Del Campo, Journal of the American Chemical Society 2011, 133, 5380; b) A. del Campo, D. Boos, H. W. Spiess, U. Jonas, Angewandte Chemie-International Edition 2005, 44, 4707).

Photocleavage of compound 2',7-bisNvoc-PTX was expected to yield the mono-substituted species 2'-Nvoc-PTX and 7-Nvoc-PTX as intermediates that can be further photolyzed to afford free PTX. In fact, HPLC analysis (Fig. 1) confirmed the presence of 2'-Nvoc-PTX (retention time, r.t.= 16.7 min) and 7-Nvoc-PTX (r.t. = 18.2 min) together with the starting compound 2',7-bisNvoc-PTX (r.t. = 21.3 min) and the fully deprotected PTX (r.t. = 11.1 min). The ratio of these compounds within the composition of the irradiated mixture changes with the exposure dose. Fig. 2 and Table B below show the compositional evolution of the mixture with increasing irradiation time as obtained by HPLC analysis. The photocleavage of the carbamate at position 7 seems to be more effective than at position 2' and higher amounts of 2'-Nvoc-PTX than of 7-Nvoc-PTX were present in the mixture at all irradiation times. This result suggests that Nvoc protected cyclic hydroxyls may be cleaved more effectively than linear ones. However, the influence of other structural factors in the photolytic mechanism cannot be ruled out. Compound 7-Nvoc-PTX could also be isolated from the irradiated mixture in milligram scale.

**Table B. Molar composition of a solution of 2',7-bisNvoc-PTX (1 mM, 5% water in acetonitril) after different irradiation times, as determined by analysis of HPLC graphs (Fig. 2)**

| **Irradiation Time (min)** | **PTX (%)** | **2'-Nvoc-PTX (%)** | **7-Nvoc-PTX (%)** | **2',7-bisNvoc-PTX (%)** |
|---|---|---|---|---|
| 0 | 0 | 0 | 0 | 100 |
| 60 | 0 | 6.3 | 3.2 | 90.5 |
| 270 | 1.7 | 17.4 | 8.6 | 72.3 |
| 450 | 6.0 | 27.6 | 15.3 | 51.1 |
| 570 | 10.6 | 32.3 | 14.9 | 42.2 |
| 735 | 18.5 | 35.6 | 18.4 | 27.5 |
| 820 | 21.2 | 38.1 | 16.4 | 24.3 |
| 1585 | 60.5 | 25.6 | 7.8 | 6.1 |

### 3. Cell culture

Human cervix carcinoma cells, HeLa cells (#ACC57, DMSZ, Germany), were cultured in DMEM medium (Invitrogen) supplemented with 10% fetal calf serum (FCS, Gibco) in a humidified incubator at 37°C/5%CO₂.

### 4. Cell vitality assay

In order to evaluate the bioactivity of the caged PCT derivatives, their cytotoxicity was tested *in vitro* using HeLa cells. Cells were incubated with PTX and caged PTX derivatives at concentrations between 0.01 and 10 µM.

The effect of PTX and derivatives on cell vitality was measured in a combined assay determining cytotoxicity and cell number by propidium iodide (PI) staining. HeLa cells (2x10⁴ cells/well) were diluted in cell culture medium (DMEM, 10 % FCS) and seeded in 96 well-plates (black plate, clear bottom, corning, Amsterdam, Netherlands). The culture medium was replaced after ~16h by compound supplemented medium (200 µL, DMEM, 10 % FCS, 1 % DMSO) or medium without compound (DMEM, 10 % FCS, 1 % DMSO) as a specific control. After 48 h, dead cells with permeable cell membranes were stained with PI (30 pg/mL, 15 min at 37°C, 5% CO₂) and the PI fluorescence intensity was measured with a fluorescence plate reader (excitation wavelength 530 nm, emission wavelength 620 nm, Tecan Infinite M1000, Austria). Subsequently, the cells were lysed with 2% Triton X-100 diluted in DMEM (0.3% Triton X-100 per well, 15 min at 37°C, 5% CO₂ and the PI fluorescence was measured again. The vitality of the cells was calculated by the ratio of dead cells (DC) before lyses and the total cell number (TCN) after lyses (1-DC/TCN).

Fig. 3: Evaluation of the cytotoxicity of PTX derivatives vs. concentration, c. The vitality of the cells (%) is the ratio between dead cells and the total cell population per growing area. A) The vitality of the three caged PTXs derivatives is compared to free PTX. B) The vitality of cells after incubation with irradiated solutions of 2',7-bisNvoc-PTX containing different amounts of uncaged (free) PTX. The ratio of PTX derivatives present in the irradiated solutions can be read out from Figure 1 and is specified in Table B.

Fig. 3A shows the vitality for the PTX derivatives at different concentrations. In the positive control, PTX significantly reduced cell vitality at concentration 0.1 µM. This value agrees with reported cytotoxicity levels by other groups (Ceruti et al., Journal of Controlled Release 2000, 63, 141). Interestingly, 7-Nvoc-PTX and 2',7-bisNvoc-PTX did not show any cytotoxicity at concentrations at least up to 10 µM, indicating the cage group effectively inhibited the activity of the drug. 2'-Nvoc-PTX showed a ~25% reduction in vitality at 0.5 µM, indicating a reduced but still significant cytotoxicity in spite of the caged 2' position. The vitality results demonstrate that 2'-Nvoc-PTX can only be regarded as caged PTX over a very narrow concentration range (<0.1 µM), whereas the new derivatives worked over the whole range of concentrations tested (up to 10 µM).

Fig. 3B shows the vitality of HeLa cells treated with defined concentrations of irradiated solutions of 2',7-bisNvoc-PTX. The irradiated solutions contain a mixture of 2',7-bisNvoc-PTX, free PTX, and the 2'- and 7-caged derivatives in different ratios (see Fig. 2 and Table B for the precise composition). Cytotoxic effects were visible already at the shortest irradiation time, indicating light-triggered release of cytotoxic PTX and 2'-Nvoc-PTX. The concentration at which cytotoxic effects appear decreases with increasing irradiation time, as expected from a higher concentration of free PTX and 2'-Nvoc-PTX in the irradiated solutions. In fact, vitality levels of 0.5 were reached for the longer irradiation times, in accordance to the values observed for PTX solutions at similar concentrations. These results confirm the presence of functional uncaged PTX in the irradiated solutions, as they were identified by HPLC (Fig. 1) and demonstrate the possibility of a light-control delivery of PTX using the present caged PTX derivatives.

### 5. Live cell imaging experiments

In order to visualize the influence of the PTX derivatives in the microtubule network of the cell, a live cell microscopy analysis of HeLa cells after incubation with 10 µM PTX derivatives for 24 hours was performed. The microtubules were stained with Tubulin Tracker, a commercial available fluorescent PTX derivative that is expected to bind to microtubules if these were not competitively blocked by the caged PTX. Fig. 4 shows the results of this analysis.

Specifically, microtubules and nuclei of HeLa cells were fluorescently stained with Tubulin Tracker (Invitrogen) and DRAQ5 (biostatus, United Kingdom) respectively, and imaged by a Leica TCS SP5 confocal laser scanning microscopy (CLSM). 30x10⁴ HeLa cells were seeded in 8-well chamber slides (Lab-Tek, nunc, Germany) and the culture medium was replaced after ~16 h by compound supplemented medium (250 µL, DMEM, 10% FCS, 1% DMSO) or medium without compound (DMEM, 10% FCS, 1% DMSO) as a specific control. After 24h, cells were carefully washed with Hank's Buffered Saline Solution (HBSS, with Ca²⁺ and Mg²⁺, Invitrogen) and the buffer was replaced by a staining solution containing 250 nM Tubulin Tracker (Invitrogen) and 5 µM DRAQ5. The cells were stained for 30 min at 37°C/5% CO2 in a humidified incubator. The staining solution was replaced by HBSS and the cells were directly imaged with a 100x plan apochromat (1.4 NA) oil immersion objective on the CLSM system equipped with a CO₂ incubator and a temperature control. Tubulin Tracker was excited with an argon laser (488 nm) and the emission was detected by 500-580 nm. DRAQ5 was excited with a HeNe laser (633 nm) and the emission was detected by 650-730 nm. The images have a voxel height and width of 90 nm. Bright field images grabbed with an Olympus IX-70 inverted microscope with a 20x phase contrast objective. Image processing performed with Leica LAS AF lite and ImageJ (Fiji).

Fig. 4: Bright field (20x) and fluorescence CLSM images (100x) of living HeLa cells treated with 10 µM PTX derivatives for 24 h. Microtubules were stained with Tubulin Tracker (green) and cell nuclei or chromosomes were stained with DRAQ5 (blue). (A, B) negative control, (C, D) PTX, (F, G) 2'-Nvoc-PTX, (H, I) 7-Nvoc-PTX, (J, K) 2',7-bisNvoc-PTX. Then microtubules were stained 30 min at 37°C/5% CO₂ with Tubulin. The diffuse stained spheres in 2'-Nvoc-PTX were probably dead cells incidentally stained by Tubulin Tracker with could not bind to the microtubules due to blocking by the substances.

In the negative control (no PTX), cells attached to the culture plate and formed a monolayer (Fig. 4A) and showed an intact filamentous microtubule network (Fig. 4B). In the positive control with PTX, the mitosis of HeLa cells was blocked, as indicated in the microscopy image by the spherical shape of the cells on the culture well (Fig. 4C) and the mitotic chromosomes arrested in the late prophase/metaphase (Fig. 4D). The absence of the mitotic spindle stained with Tubulin Tracker indicated the blocking of the microtubule binding sites by the PTX.

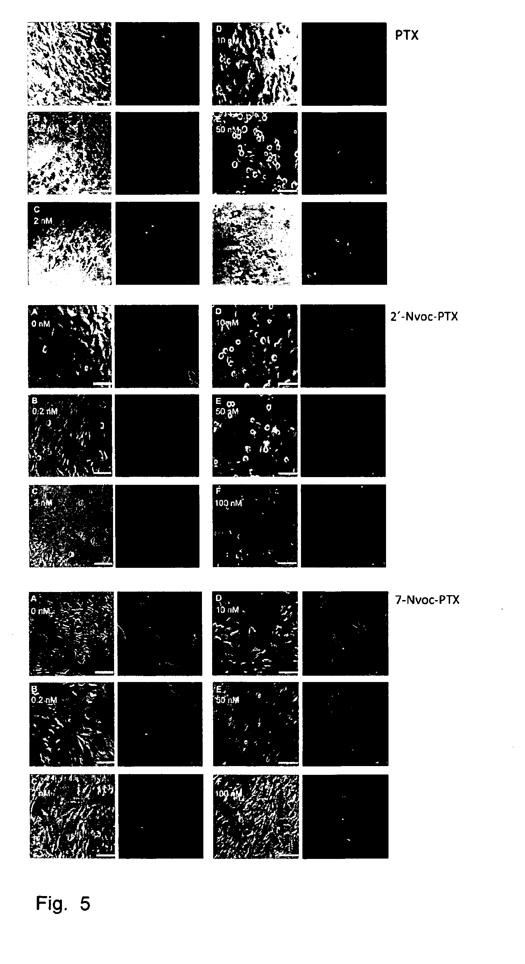

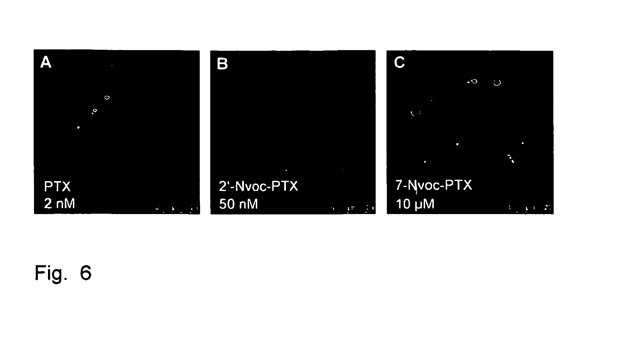

Cells treated with 2'-Nvoc-PTX showed similar results to free PTX, in agreement with the results from the vitality assay (Fig. 4E,F), and confirming that this derivative is cytotoxic in spite of containing the cage at the 2' position. In contrast, 7-Nvoc-PTX treated cells formed an adherent monolayer similar to the negative control (Fig. 4G), but the fluorescence image showed important differences (Fig. 4H). The microtubules were less stained, tripolar spindles appeared and the cells developed multiple nuclei with different sizes. The microtubule staining was concentrated in the proximal area of the nucleus/nuclei suggesting that 7-Nvoc-PTX binds more distal to the (+) ends where new β-tubulin subunits polymerize. It is known that low concentrations of PTX in cell culture can trigger beside mitotic arrest and cell death, unequal cell divisions to produce aneuploid daughter cells. The present inventors hypothesized that the multiple tripolar spindles and multiple nuclei observed for 7-Nvoc-PTX occurred due to a strongly reduced β-tubulin binding activity comparable the effects of PTX at low doses. In fact, dose-response curves for lower concentrations (0.2-100 nM) of PTX (Fig. 5), 2'-Nvoc-PTX and 7-Nvoc-PTX revealed that 2 nM PTX trigger a nuclei pattern similar to 50 nM 2'-Nvoc-PTX and 10 µM 7-Nvoc-PTX (Fig. 6). These results demonstrate that a single cage at 7 position is not enough to completely inhibit bioactivity of PTX and point out the relevance of the analysis of the microtubule network to complement the standard cytotoxicity assays in order to meaningful evaluate the effects of PTX prodrugs. However, the 7 position seems to be more relevant than the 2'position for β-tubulin binding, since 7-Nvoc-PTX prevented mitotic blocking (~ 200 fold) more effectively than 2'-Nvoc-PTX at the concentration tested (10 µM).

The microtubule network of HeLa cells treated with 2',7-bisNvoc-PTX were evenly stained by Tubulin Tracker and showed similar patterns than the negative control (Figure 4I). These results agree with the vitality tests and indicate that the double substitution at 2',7-bisNvoc-PTX effectively inhibited binding of PTX to β-tubulin (Figure 4J).

Summarizing, disclosed herein are the synthesis and properties of three different variants of caged Paclitaxel that allow light-driven dynamic regulation of PTX activity and, therefore, of microtubule growth, mitotic arrest and eventually cell dead. In particular, a double caged PTX has been synthesized and characterized containing two chromophores attached to the 2' and 7' positions. This caged PTX was used at concentrations up to 10 µm in in-vitro cultures without causing any cytotoxic effects. Light exposure triggers the uncaging reaction and allows control delivery of free PTX to the solution. The present derivatives, in particular the double caged PTX derivatives, represent a valuable molecular tool for cell and molecular biologists interested in controlling microtubule assembly and can be advantageously used in medical applications, e.g. in pharmaceutical compositions for photodynamic therapy of cancers, where the drug will only be liberated at "illuminated" tissue.

## Claims

1. A photoactivatable paclitaxel derivative wherein at least the C7 position of the paclitaxel molecule is substituted with a photocleavable moiety.

2. The paclitaxel derivative according to claim 1, which is disubstituted at the C7 and C2' positions with photocleavable moieties which may be the same or different.

3. The paclitaxel derivative according to claim 1 or 2, wherein the photocleavable moiety or moieties is/are a chromophore.

4. The paclitaxel derivative according to claim 3, wherein the chromophore is selected from the group comprising
- 2-nitrobenzyl ethers, carbamates or carbonates and their 4,5-dialkoxy (nitroveratryl) substituted variants
- 2-nitrophenylethyl ethers, carbamates or carbonates and their 4,5-dialkoxy substituted variants, or their p-substituted derivatives
- α-carboxy-2-nitrobenzyl ethers, carbamates or carbonates and their 4,5-dialkoxy substituted variants
- 2(2-nitrophenyl)ethyl ethers, carbamates or carbonates and their 4,5-dialkoxy substituted variants
- coumarin-4-ylmethyl ethers, or carbonates and their 7-alkoxy, 6,7-dialkoxy, 6-bromo-7-alkoxy or 7-dialkylamino substituted variants
- p-hydroxyphenacyl ethers or carbamates and their meta-substituted derivatives
or structurally related compounds.

5. The paclitaxel derivative according to claim 4, wherein the chromophore is 4,5-dimethoxy-2-nitrobenyloxycarbonyl (Nvoc) or a structurally related compound.

6. The paclitaxel derivative according to claim 5 which is 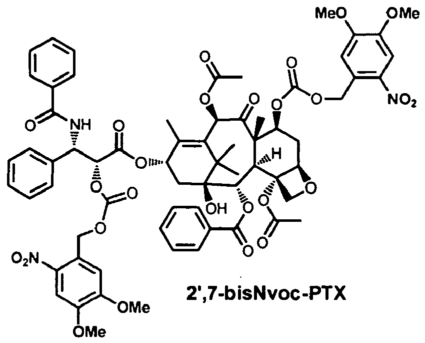 or

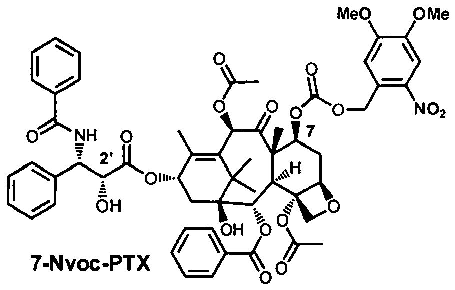

7. The paclitaxel derivative according to any one of claims 1-6, which has no cytotoxicity up to a concentration of at least 10 µM.

8. The paclitaxel derivative according to any one of claims 1-7, having a reduced ß-tubulin binding activity which is not more than about 1 %, preferably about 0.1%, of the activity of underivatized paclitaxel, in particular not more than about 0.02% for the 7-substituted or 0.002% for the 2', 7-bisubstituted derivatives.

9. The paclitaxel derivative according to any one of claims 1-8, wherein the photo-cleavable moiety is cleavable by means of irradiation with light having a wave length > 320 nm, in particular in the range from 320 to 500 nm for single photon excitation processes and > 500 nm for multiphoton excitation.

10. Use of the paclitaxel derivative according to any one of claims 1-9 in biological, medical and pharmaceutical applications.

11. The use according to claim 10 for microtubule stabilisation and affecting and/or monitoring/detecting the interaction between motor proteins and other microtubule associated proteins, or for directing microtubule growth and microtubule-related molecular transport in bionanotechnology.

12. The use according to claim 10 for preparing a medicament for treating tumors, in particular in photodynamic tumor therapy.

13. The use according to claim 10, wherein the tumors are solid tumors, such as metastatic breast cancer, head and neck carcinomas, ovarian carcinoma, non-small cell lung cancer, melanoma, Kaposi's sarcoma etc.

14. An antitumor medicament comprising the paclitaxel derivative according to any one of claims 1-9.

15. The antitumor medicament according to claim 14 which is for treating solid tumors, such as metastatic breast cancer, head and neck carcinomas, ovarian carcinoma, non-small cell lung cancer, melanoma, Kaposi's sarcoma etc.
